# EUROPEAN PATENT APPLICATION

(11) **EP 0 526 166 A2**
(43) Date of publication of application: **03.02.1993**
(21) Application number: 92306883.7
(22) Date of filing: 28.07.1992
(51) Int. Cl.: G06F 15/42

(54) **Patient compliance monitoring method and system**

(30) Priority: 29.07.1991 US 737351
(71) Applicant: Dessertine, Albert L., Flemington, New Jersey 08822 (US)
(72) Inventor: Dessertine, Albert L., Flemington, New Jersey 08822 (US)
(74) Representative: Ablewhite, Alan James

(57) **Abstract**

The present invention is directed to a method of monitoring a patient's medicine compliance. It involves utilizing an automatic compliance monitoring device which tracks patient compliance data, along with a wave energy transmitter and power source, all connected to a medicine container. A receiver is connected to a computer with a display unit. The compliance monitoring device or the computer is programmed to calculate compliance requirements of the container e.g. by number of cap openings, by dispensing count or by weight information obtained by the automatic compliance monitoring device, for each dosage administration for the prescription period. The wave energy transmitter on the medicine container continuously, periodically, or randomly transmits raw or calculated data to the receiver and from there to the computer to compare actual usage with compliance required, to determine compliance results on the display unit to permit compliance monitoring on a monitor at a remote location. Optionally, other patient characteristics are also monitored and feedback is provided. The present invention includes both the method and the system of interconnected devices to practice the method as well as a single component which includes in combination, the medicine container, compliance device, transmitter and power source. Feedback may be to any professional or any remote location, and a single medicine or a plurality of medicines may be monitored.

## Description

The present invention is directed to a method, system and component for monitoring a patient's compliance with a medicine regimen. It is directed to compliance monitoring by another person other than the patient, such as a relative or a professional such as a pharmacist, doctor, hospital, clinic or the like, at a location remote from the patient. The present invention involves the use of one or more automated compliance monitoring devices, wave energy transmitters, remote receivers and computers for monitoring and optionally includes monitoring of other patient status information such as physical characteristics, e.g. heart rate, blood pressure, etc. and/or treatment program compliance such as physical therapy or exercise regimens.

The use of precision, automatic compliance monitoring devices, unique programming and remote radio transmission of data directly from the medicine containers to computers and linking peripherals to monitor medicine regimen compliance has not been taught in the prior art.

U.S. Pat. No. 4,577,710 issued to Edward Ruzumno is directed to an apparatus for promoting good health which involves a personal weight scale and an information and message center which may be used merely for weight control or may be used for specific messages pertaining to a health condition with pre-taped feedback from a physician. This recently issued patent represents the concept of patient weight monitoring for general or specific health purposes. However, it does not pertain to medicine regimen compliance, automatic compliance monitoring devices or computer linking as in the present invention.

Most recently, Time Magazine, June 5, 1989, at page 70 reported that Aprex, a California Company has developed a cap for use with medication containers. A computer chip in the cap records the day and time each time the cap is opened (for taking medicine). The professional, e.g. the doctor will, at a later time or visit, put the cap into an electronic analyzer that lets the user know how regularly the medicine was taken. This automatic compliance monitoring system, unfortunately, does not permit the doctor or pharmacy to monitor unless the cap is delivered to the analyzer.

U.S. Patent No. 4,899,839 issued to Dessertine and Hudson and assigned commonly to the assignee herein, describes a compliance system using a scale tied into a computer for weighing medicine containers and calculating usage, and, therefore, compliance, at specific or random times during a prescription period. This system requires the patient to physically place the medicine container on a scale and possibly perform other tasks related thereto.

U.S. Patent No. 5,016,172 to the inventor herein, Dessertine, and commonly assigned, describes compliance monitoring systems where the medicine container has a device for tracking (collecting, storing, retrieving) compliance data, and then inputting same by connecting the container to a computer, for compliance monitoring. Again, this requires some action on the part of the patient, e.g. plugging the computer and container together.

The present invention uniquely enables doctors, hospitals, pharmacies, manufacturers, data centers, etc. to monitor compliance from remote locations without requiring the patient to take any specific action not needed in the absence of compliance. In other words, the present invention is the first patient passive compliance monitoring method and system that provides the opportunity for ongoing feedback/information on the patient's compliance activity.

The present invention is directed to a method of monitoring a patient's medicine compliance. It involves utilizing an automatic compliance monitoring device which tracks patient compliance data, along with a transmitter and power source, all connected to a medicine container. A receiver is connected to a computer with a display unit. The compliance monitoring device or the computer is programmed to calculate compliance requirements of the container e.g. by number of cap openings, by dispensing count, by light band or intensity measurement, by weight information obtained by the automatic compliance monitoring device or the like, for each dosage administration for the prescription period. The radio transmitter on the medicine container continuously, periodically, or randomly transmits raw or calculated data to the receiver and from there to the computer to compare actual usage with compliance required, to determine compliance results on the display unit to permit compliance monitoring on a monitor at a remote location. Optionally, other patient characteristics are also monitored and feedback is provided. The present invention includes both the method and the system of interconnected devices to practice the method as well as a single component which includes in combination, the medicine container, compliance device, transmitter and power source. Feedback may be to any professional or any remote location, and a single medicine or a plurality of medicines may be monitored.

The present invention, its advantages and objects will be more fully understood when the specification herein is taken in conjunction with the drawings appended hereto, wherein:
Figure 1 illustrates a diagrammatic representation of some preferred embodiments of the present invention;
Figure 2 illustrates an alternative embodiment diagrammatic representation of the present invention;
Figure 3 illustrates a schematic diagram of the present invention showing the interrelationships of the various components; and,
Figure 4 shows an alternative embodiment schematic diagram of the present invention.

The present invention involves a method and a system for monitoring a patient's compliance with regard to adhering to a particular medicine regimen as well as a specific component involved in the system. It is directed to compliance monitoring by someone at a remote location, e.g. a relative or friend or paid service or by a professional such as a pharmacist, a doctor, a physical therapist, a chiropractor or the like or by a hospital or clinic or staff of a convalescence home or a remote data collection center, a government agency or other party or any combination of these. Thus, the present invention involves remote monitoring independent from the patient. Further, the invention involves using one or more automatic compliance monitoring devices, wave energy transmission, e.g. radio transmission, from the medicine container to a receiver and then, directly or indirectly, to one or more computers which are interconnected. In some embodiments the receiver may be located within the computer or attached integrally to it. In addition to medicine regimen, optional status of patient health characteristics may be monitored with computer tracking, feedback and other communication. Thus, a patient's heart rate, blood pressure, glucose level, cholesterol level, weight or other physical characteristics may be monitored or some compliance with a physical therapy program or exercise program may be monitored in addition to the medicine regimen monitoring.

By "medicine container" used herein is meant any portable container which may be carried by a patient and has a plurality of dosages of medicine. The medicine itself may be liquid, slurry, cream, paste, powder, capsule, caplet, tablet or any other dosage capable form. The medicine container will necessarily have a cap portion and a base portion. The "cap portion" may be a fully removable cap, a connected cap such as a hinged cap, or merely the door of an orifice. The "base portion" is the container minus the cap portion and is that part which holds the medicine.

The "automatic compliance monitoring device" described herein is meant to be any known or to be developed device connected to a medicine container - pill, capsule, liquid or otherwise - which tracks (recognizes and stores) data pertaining to actual medicine consumption or container usage. "Actual medicine consumption" means actual amount of medicine taken from a medicine container. Such devices may include cap opening counting devices such as the Aprex device described in the prior art above, or automatic pill counting devices or light band-based or light intensity-based contents measuring devices or weight or volume tracking devices connected to a container. It may be an optical scanner which measures volume or fill level or it may be another container-attached or attachable device. The details of the workings of such devices are now within the skill of the artisan and variations may be made without exceeding the scope of the invention.

The "transmitter" herein is a wave energy transmitter such as a radio or microwave transmitter, although no frequency limit to the transmission is intended. It has a power source for transmitting and may be capable of transmitting only short distances or long distances depending upon costs and purpose. For example, a transmitter in a medicine container for a hospital patient may need only a very short transmission with receivers in every room, on every floor or centrally located. Or, the user may have a transmitter at home or at work. The transmitter may be set up to transmit continuously, periodically or randomly, e.g in response to a stimulus. The transmitter may send unique identifiers and then compliance data, e.g. social security number of patient, medication code and pills removed , or weight, or fill level. The power source may be the same as one running the compliance device and may be, e.g. solar chargeable.

The receiver used may be any which will perform the function of receiving the data transmitted and forwarding it. The receiver may, for example,
(a.) forward the data directly to one or more computers;
(b.) pass the data through telephone lines or cable television lines to one or more data receiving computers at a more remote location;
(c.) pass the data to an amplifier, second transmitter where it may be retransmitted to a remote receiver directly or even via satellite to a regional, national or international data center;
(d.) simulcast the data by any or all of the above or any other available methods to diverse receivers, including conversion of data from radio to electronic, digital, sonic, optical or printed format;
(e.) in addition to, or separate from, any of the above, forward compliance or non-compliance information to a computer with a displayed message, e.g. with audio signal, to the patient to advise the patient to take some corrective action as needed. Likewise, unique computer display of problem compliance to a doctor, hospital, agency, etc. may be effectuated by the present invention method.

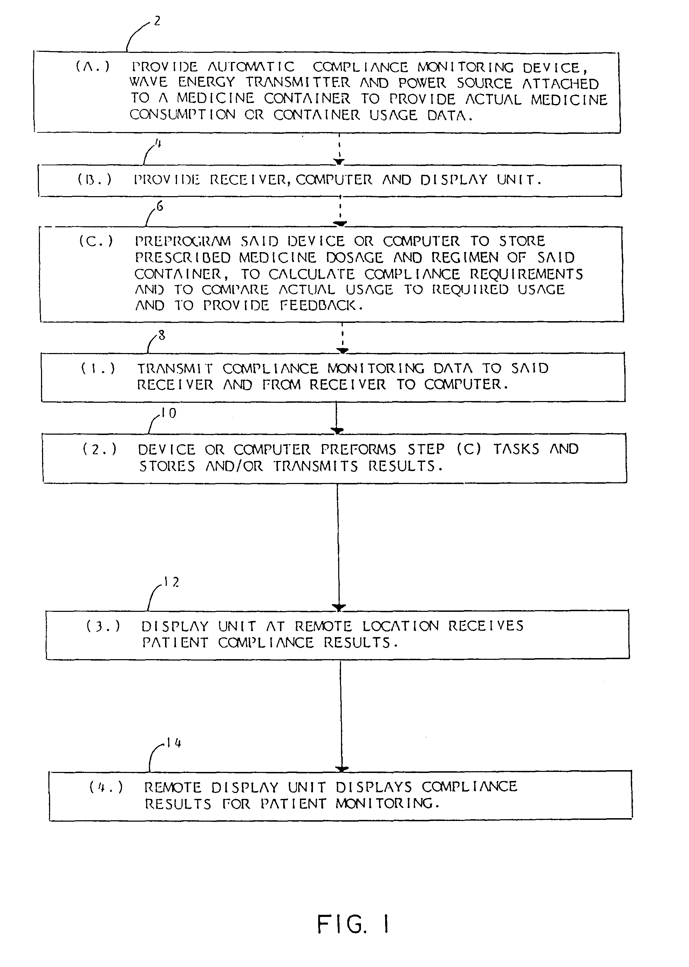

Referring now to Figure 1, there is shown a flow diagram with steps (A), (B) and (C) shown which involve preparing the system for using the method of the present invention as well as steps 1 through 4 which show an embodiment involving the steps of the present invention. Thus, frame 2 shows step (A) wherein an automatic compliance monitoring device, a wave energy transmitter, e.g. radio transmitter, and power source are attached to a medicine container. The frequency of the wave energy transmitter is not critical as long as the wave energy is sent and received in useable form. Low, high, ultrahigh, microwave, C-band, Ku-band or any available frequency may be used.

Frame 4 indicates in step (B) that, separately, a receiver, computer and display unit are provided. Thus, any automatic compliance monitoring device which is capable of accurately, electronically determining or recognizing medicine consumption and/or container usage data and recording and storing same is adapted for transmitting information to a receiver and computer. The computer may be active or passive, i.e. do significant work with the data or simply convert it so it may be transmitted to and displayed on a remote display unit. Thus, for example, the device may be used to generate a signal which, after transmission and reception, may be amplified and then converted from analog to digital using a state of the art analog digital converter and then the signal would be used to perform compliance calculations on the computer. Additionally, frame 6 of Figure 1, step (C) involves the pre-programming of the device or the computer to store prescription requirements, to recognize various information such as indicated and to determine or calculate compliance requirements and to compare these to actual data. The system may involve storing sequential information e.g. dosage frequency data, so as to determine a required regimen and to compare actual usage against the required usage on a periodic basis or at random times, and to provide feedback. Having thus preprogrammed the device and/or the computer to perform these functions as well as optional functions described below, the system may now be used to practice the method of the present invention.

Referring again to Figure 1, frame 8 shows the first step (1) wherein data produced by an automatic compliance monitoring device from a medicine container is transmitted to said receiver and computer at least from time to time. As used herein, "from time to time" may be after each dosage is taken or at set intervals or at random intervals. Transmission could, alternatively, be continuous. Frame 10 shows the second step wherein the device or computer performs the functions preprogrammed per step (C), frame 6, discussed above. The computer recognizes the difference between the actual data and the dosage requirements of a patient after it calculates the required usage based on the dosage amount and frequency. Frame 12 shows the third step wherein a remote display unit receives the results of the compliance comparison, and frame 14 shows the fourth step wherein the computer displays compliance results for patient monitoring at a remote location.

As can be seen, the computer used in the present invention may be any conventional computer system and may be interlinked by way of modem or radio transmission or any other computer linking possibilities.

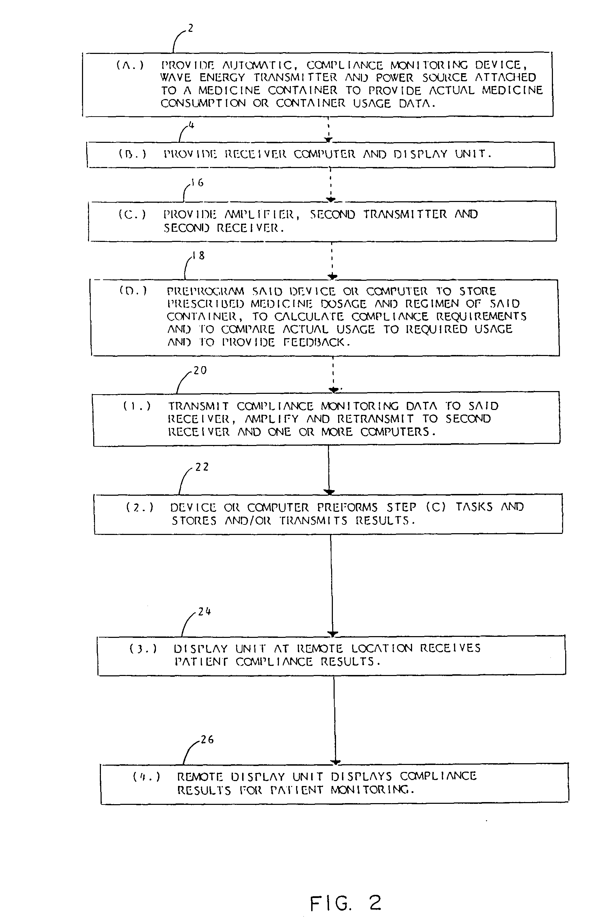

Figure 2 illustrates another diagrammatic representation of a present invention system. Here, like frames to Figure 1 are like numbered and are identically described. In this embodiment, however, as shown in frame 16, step (C) calls for providing an amplifier, second transmitter and second receiver. In frame 18, programming is as in frame 6 of Figure 1.

In the first step (1) shown in frame 20 of Figure 2, the data is transmitted, received, amplified and re-transmitted to a second receiver and then to one or more computers. Thus, a small, battery powered transmitter may transmit data to a remote but local receiver, which may then boost the data by amplifying same and re-transmitting to another receiver and then to one or more computers. Steps (2), (3), and (4) shown by frames 22, 24 and 26 of Figure 2 illustrate the same steps, (2), (3) and (4) as shown in Figure 1.

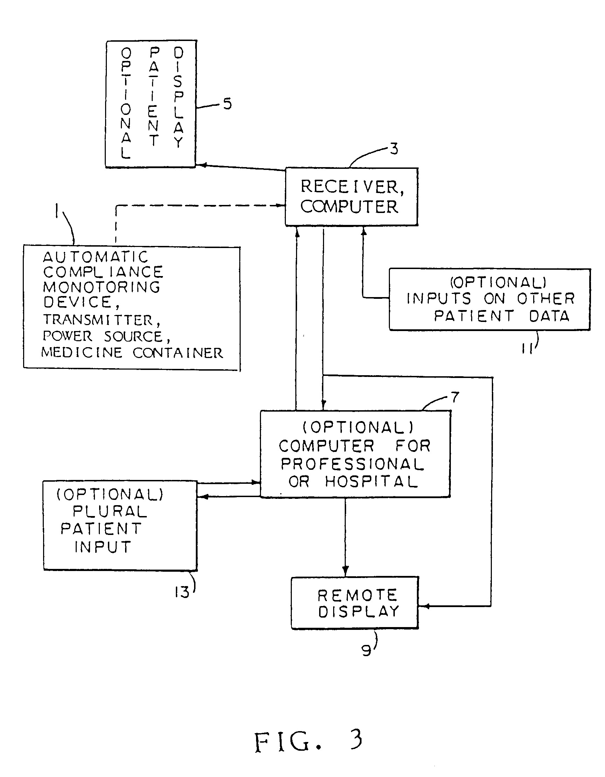

Referring now to Figure 3, there is shown a component 1 having an automatic compliance monitoring device, radio transmitter, power source and medicine container. The device provides data to the transmitter in radio transmission form and this is transmitted to receiver/computer 3 which is in turn connected to remote display unit 9. Additionally, receiver/computer 3 is connected to an optional, remote computer for professional or hospital use shown as 7 and when utilized this is connected to remote display unit 9. Optionally, inputs on other patient data may be fed into receiver/computer 3 and these optional inputs are shown as block 11. Also, optional patient display unit 5 may be connected to the system for automatic feedback and/or instructions from a professional who is monitoring the patient's medicine regimen. Further if a professional or hospital is monitoring patient's medicine regimen, then computer 7 and remote display unit 9 may be interconnected with a plurality of other patient inputs 13 and thus professionals may sequentially or randomly monitor a plurality of patients with the same system. This may be beneficial for convalescent homes, cancer treatment centers, rehabilitation centers and the like where patient progress and medicine regimen may be monitored from a single computer station, as well as to other data collection centers.

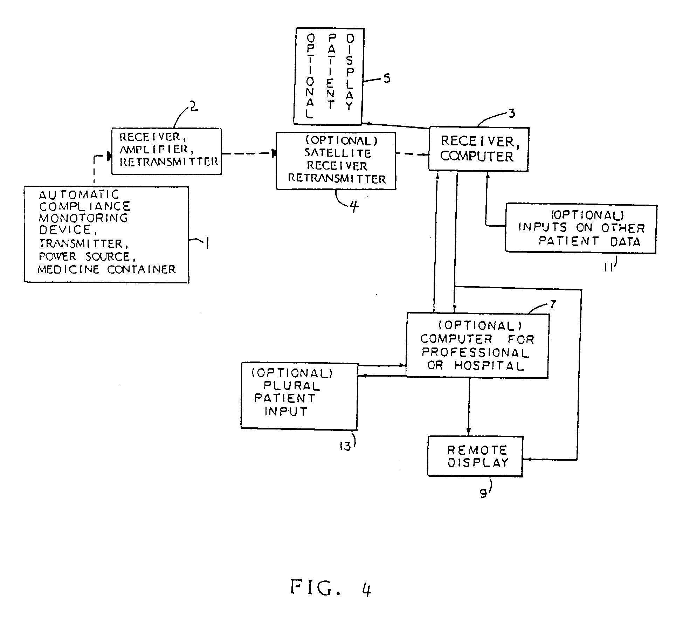

Figure 4 illustrates another schematic diagram of an alternative embodiment of the present invention. Most of the block frames shown are the same as those shown in Figure 3 and like parts are like numbered. However, in this embodiment, the transmitter from component 1 sends data to component 2 which includes a receiver, amplifier and re-transmitter. The re-transmitter forwards data by transmitting to component 3. For example, component 2 may be located in a home, in a hospital room, at a radio tower or close to or a significant distance from component 1 depending upon the power of the component 1 transmitter. Subsequent functions are similar to those described in conjunction with Figure 3 above.

An optional feature involves the use of satellite reception and re-transmission shown as component 4 in Figure 4. In other words, the transmitter on the medicine container may transmit to a booster transmitter which may then transmit to as many receiver re-transmitter set ups as desired, with appropriate amplification as needed.

In one preferred embodiment of the present invention, the medicine taker or user of the system may trigger an automatic transmission upon opening the container to input with the device and the computer each time the medicine is taken. Thus, in this embodiment, the expression relating to providing input from the device to transmit to the receiver and the computer "from time to time" refers to each time it is used. In this manner, optimum monitoring is achieved so that if there is an overdosage or a missed dosage, the present invention system will alert the user, or a professional or both of the deficiency or overdosage. Additionally, the system may, as mentioned, be used to provide monitoring for more than one medication at a time. This may be done by including an optional identifier whereby the compliance monitoring device for each medication has a code which is transmitted with the compliance data and read by and properly identified by the computer before the computer calculates and compares actual and required dosages. Alternatively, other ways of discerning advice for one medication from another may be included without exceeding the scope of the present invention. In another preferred embodiment of the present invention, the system is used to also monitor heart rate, blood pressure, glucose, cholesterol, blood cell count, respiration, body weight or other physical characteristic or characteristics and these may be monitored by the patient or by a professional. Further, or in addition to the foregoing, the system may be used to also monitor compliance with a physical regimen such as a physical exercise program or a physical therapy program. This may be done by having a user active system wherein the user must feed in information to a transmitter for transmission to the receiver and the computer each time an exercise or a therapy session occurs or, more preferably, the system itself may be interlinked with actual exercise equipment such as tread- mills, exercise bicycles, rowing machines, weight pulls, etc. and direct information from the exercise equipment will be communicated to the computer via one or more transmitters and receiver(s) and subsequently to the monitor to assure compliance by the patient.

In any of the above embodiments, the present invention in its more refined embodiment may include computer recognition and feedback of actual times and dates for every required dosage (specific day and/or time of day) and for every actual dosage removed from the medicine container. In other words, the system will provide the patient and/or professional with output showing actual versus required usage on a time based dosage comparison. When this mode is utilized, the computer will be provided with necessary information to generate requirements and the patient may input the computer with data from the compliance monitoring device of the medicine container at each use of the medicine container.

While the computer described above is generally preprogrammed to receive data from the automatic compliance monitoring device radio transmission and to process that data, the computer may be more complex or less complex without exceeding the scope of the present invention. For example the computer may be merely a unit that reads electronic information provided to it and converts it for transmission to a display unit. Alternatively, the computer may include any or all of the previously mentioned comparison and reporting functions and optional functions any may also include means for a patient to provide subjective information back to a hospital or doctor such as how the patient feels or how the patient is responding to the medication.

Obviously, numerous modifications and variations of the present invention are possible in light of the above teachings. It is therefore understood that within the scope of the appended claims, the invention may be practiced otherwise than as specifically described herein.

## Claims

1. A method of monitoring a patient's medicine compliance which comprises:
(a) providing an automatic compliance monitoring device, which device automatically tracks at least one type of patient medicine compliance data, said device being connected to a medicine container;
(b) providing a wave energy transmitter and a power source to drive said transmitter for transmission of said patient medicine compliance data to a remote location, said transmitter being electronically connected to said automatic compliance monitoring device for said transmission and said data transmitter and power source being connected to said medicine container;
(c) providing a receiver at said remote location and providing a computer to which said receiver inputs patient medicine compliance data;
(d) at least from time to time, transmitting patient medicine compliance data from said transmitter to said receiver and computer;
(e) having either said device or said computer programmed to store the prescribed medicine dosage and regimen of said container;
(f) having either said device or said computer programmed to calculate compliance requirements for each dosage administration for the prescription period of the medicine and for comparing the actual medicine consumption or container usage with the compliance requirements;
(g) connecting said computer to a display unit at a remote location away from the automatic compliance monitoring device; and,
(h) visually displaying the compliance results on said display unit to permit compliance monitoring.

2. The method of claim 1 wherein two computers are used, a first computer being located so as to be readily available to receive raw data from said automatic compliance monitoring device and a second being remotely located with said display unit.

3. The method of claim 2 wherein said first computer includes a display unit to provide corrective instructions to a patient when appropriate.

4. The method of claim 3 wherein said first computer is programmed to provide specific day and/or time of day information to display compliance information to a patient on its display unit.

5. The method of any of claims 1 to 4 wherein said transmission is to a professional for compliance monitoring.

6. The method of any of claims 1 to 5 wherein the method is repeated for a plurality of different containers of medicine with different automatic compliance monitoring devices and the computer determines and stores compliance information for each of said plurality of different devices.

7. The method of any of claims 1 to 6 wherein said automatic compliance monitoring device, said radio transmitter and said power source are located in a cap portion of a medicine container.

8. The method of claim 7 wherein said automatic compliance monitoring device is a cap removal counting device.

9. The method of claim 7 wherein said said automatic compliance monitoring device is a weighing device.

10. The method of and of claims 1 to 6 wherein said automatic compliance monitoring device, said radio transmitter and said power source are located in a base portion of a medicine container.

11. The method of claim 10 wherein said automatic compliance monitoring device includes an optical scale feature to measure medicine container content.

12. The method of any of claims to 1 to 11 wherein said receiver is located in a first remote area from said device and said computer is located at a second remote area from said device and said receiver and computer are connected to one another by additional wave energy transmission.

13. A system for patient medicine compliance and patient status monitoring, which comprises:
(a) a medicine container having a cap portion and a base portion;
(b) at least one automatic compliance monitoring device which automatically tracks at least one type of data related to actual medicine consumption or container usage, said device being connected to a medicine container;
(c) a wave energy transmitter and power source for transmission of patient medicine compliance data, said transmitter being electronically connected to said automatic compliance monitoring device and said transmitter and power source being connected to said medicine container;
(d) a receiver at a remote location from said radio transmitter;
(e) a computer connected to said receiver to receive input therefrom on patient medicine compliance data;
(f) a display unit connected to said computer to permit patient medicine compliance monitoring;
Further, wherein either said device or said computer is programmed to store prescribed medicine dosage and regimen of said container and having either said device or said computer programmed to calculate compliance requirements for each dosage administration for the prescription period and for comparing the actual medicine consumption or container usage with the com- plaince requirements to display comparison results on said remote display unit.

14. A patient medicine compliance system component, which comprises:
(a) a medicine container having a cap portion and a base portion;
(b) an automatic compliance monitoring device which atuoamtically tracks at least one type of patient medicine compliance data and being connected to said medicine container;
(c) a wave energy transmitter and power source to drive the transmitter for transmission of said patient medicine compliance data to a remote location, the transmitter being electronically connected to said automatic compliance monitoring device and the transmitter and power source being connected to said medicine container.
